# EUROPÄISCHE PATENTANMELDUNG

(11) **EP 0 649 660 A2**
(43) Veröffentlichungstag der Anmeldung: **26.04.1995**
(21) Anmeldenummer: 94116552.4
(22) Anmeldetag: 20.10.1994
(51) Int. Cl.: A61K 47/10, A61K 47/24, A61K 47/22

(54) **Pharmazeutische Zubereitung für die parenterale, enterale und dermale Verabreichung von praktisch unlöslichen Arzneistoffen und Verfahren zu ihrer Herstellung**

(30) Priorität: 26.10.1993 DE 4336434
(71) Anmelder: HOECHST AKTIENGESELLSCHAFT, D-65929 Frankfurt am Main (DE)
(72) Erfinder: Reul, Bernhard, D-61462 Königstein (DE); Petri, Walter, Dr., D-65185 Wiesbaden (DE); Winkler, Irvin, Dr., D-65835 Liederbach (DE)

(57) **Zusammenfassung**

Es wird eine Arzneistoffzubereitung enthaltend einen in Wasser und lipophilen Medien praktisch unlöslichen Arzneistoff und einen oder mehrere wasserlösliche oder in Wasser micell-kolloidal lösliche physiologisch verträgliche Ampho-Surfactant/s, die in einem oder mehreren physiologisch verträglichen, wasserfreien und wassermischbaren Lösungsmittel/n in gelöster Form vorliegen, beschrieben. Diese Arzneistoffzubereitung wird dermal, buccal oder enteral oder nach Zusatz von Wasser oder wasserhaltigen Lösungen parenteral, enteral oder buccal angewendet.

## Beschreibung

Die Erfindung betrifft eine Arzneistoffzubereitung enthaltend einen in Wasser und lipophilen Medien praktisch unlöslichen Arzneistoff und einen oder mehrere wasserlösliche oder in Wasser micell-kolloidal lösliche physiologisch verträgliche Ampho-Surfactant/s, die in einem oder mehreren physiologisch verträglichen, wasserfreien und wassermischbaren Lösungsmittel/n in gelöster Form vorliegen.

Die Arzneistoffzubereitung ist eine Lösung und wird nach Zusatz von Wasser oder wasserhaltigen Lösungen parenteral, enteral oder buccal angewendet. Die Arzneistofflösung kann aber auch ohne Wasserzusatz, d.h. wasserfrei, dermal, buccal und enteral angewendet werden.

Arzneistoffzubereitungen schwerwasserlöslicher Arzneistoffe sind bereits beschrieben worden.

EP-B-0 256 285 beschreibt zur parenteralen Injektion geeignete Zubereitungen schwerwasserlöslicher Arzneistoffe, die eine Ölphase (z.B. Fettsäuretriglycerid), einen Emulgator (z.B. Sojalecithin) und Wasser enthalten. Die Arzneistoffzubereitungen sind Suspensions-Emulsionen und werden als solche verabreicht. Aufgrund des Herstellungsverfahren und der Nicht-Zulässigkeit der i.v.-Verabreichung am Menschen sind diese Suspensions-Emulsionen nur für orientierende Wirksamkeitsuntersuchungen schwerwasserlöslicher Arzneistoffe im Tierversuch geeignet.

In EP-A-0 539 319 wird eine Arzneistoffzubereitung in Form eines Emulsionskonzentrates - aufbauend auf einer Ölkomponente, zwei Komplexemulgatoren (HLB > 10 und HLB < 4) und einer wasserlöslichen Co-Komponente (z.B. Ethanol, 1,2-Propandiol) beschrieben. In diesem Emulsionskonzentrat (sog. "micro-emulsion-preconcentrate") ist der schwerwasserlösliche Arzneistoff gelöst. Die wasserfreie Zubereitung ist nur für die orale Applikationsart (z.B. in Form von Gelatinekapseln) geeignet.

Nach DE-A-4 129 309 werden schwerwasserlösliche Arzneistoffe mit Hilfe von lipophilen Alkyl-Lactamen, nichtionischen oberflächenaktiven Substanzen und wassermischbaren, pharmazeutisch verwendbaren Flüssigkeiten gelöst. Die wasserfreie Lösung wird entweder in Gelatinekapseln oder nach Vordispergieren mit Wasser oral appliziert. Je nach Zusammensetzung der Arzneistoffzubereitung entstehen Emulsionen oder Mikrosuspensionen.

DE-A-2 730 570 betrifft Injektionslösungen von in Wasser schlecht oder unlöslichen Pharmaka auf rein wäßriger Basis in Kombination mit natürlichen Micellbildnern (z.B. Glycocholat) und Phospholipiden (z.B. Eilecithin).

Die Arzneistoffzubereitungen sind lagerstabile, wasserhaltige Fertiglösungen mit einem sehr hohen Gehalt an oberflächenaktiven Stoffen. Ein hoher Gehalt an oberflächenaktiven Stoffen ist bei einer Zubereitung für die i.v. Applikation unerwünscht.

EP-A-0 488 142 beschreibt eine lösungsmittelfreie Herstellung wirkstoffhaltiger Liposomen von schwerwasserlöslichen Arzneistoffen mit natürlichen und synthetischen Phospholipiden. Die wässrigen Liposomen-Dispersionen eignen sich für die parenterale Applikation.

EP-A-0 467 838 beschreibt die Herstellung wirkstoffhaltiger Liposomen von schwerwasserlöslichen Arzneistoffen mit rein synthetischen Phospholipiden unter Einsatz von Essigsäure als Lösungsmittel und anschließender Neutralisation. Die Zubereitung enthält Wasser und ist frei von organischen Lösungsmitteln.

Liposomen-Formulierungen sind als Fertig-Arzneimittel jedoch ungeeignet wegen der Bildung von lyso-Lecithinen (Hydrolyse der Phospholipide) und ihrer Fusionseigenschaften zu größeren Gebilden (Aggregation).

In EP-B-0 158 441 wird ein Herstellungsverfahren für wirkstoffhaltige Liposomen von wasserlöslichen, biologisch aktiven Verbindungen beschrieben. Der Wirkstoff und das hierfür geeignete Membranlipid ist, ggfls. mit membranstabilisierenden bzw. die Permeabilität fördernden Hilfsstoffen, wie z.B. Cholesterol oder Fettsäuresorbitanester, in einem mit Wasser mischbaren Lösungsmittel gelöst. Zusätzlich wird noch je nach Wirkstoff und Applikationsform eine bestimmte Menge Wasser zugegeben. Nach weiterer Zugabe von sog. Überschußwasser entstehen unter spez. Herstellungsbedingungen Liposomen mit vom Membranlipid eingeschlossenen biologisch aktiven Verbindungen.

Für die wasserhaltigen Zusammensetzungen bestehen auch hier wie für alle Liposomen- oder vesikulären Formulierungen die bereits beschriebenen Instabilitäten (z.B. Hydrolyse, Fusionstendenz). Darüberhinaus sind diese Zusammensetzungen bei Verwendung von physiologisch verträglichen Lösungsmitteln für den Einsatz von praktisch unlöslichen Arzneistoffen aufgrund ihrer geringen Lösungskraft völlig ungeeignet.

Ferner sind in EP-B-0 158 441 noch wasserhaltige und wasserfreie Zubereitungen für die Herstellung von sog. Lösungsaerosolen beschrieben. Aufgrund der Zusammensetzung der Endformulierung (= Aerosol-Formulierung) ist eine Bildung von Liposomen auszuschließen, weil die druckverflüssigbaren Fluor- Chlorkohlenwasserstoffe (F 12/F 114) das für die Liposomenbildung erforderliche Lipid lösen und damit die Liposomenbildung unterbinden.

EP-A-0 475 160 betrifft Arzneistoffzubereitungen von leicht- und schwerwasserlöslichen Arzneistoffen bestehend aus einem hochreinen Phospholipid (Phosphatidylcholingehalt > 95 %), Tensiden, Ethanol und Wasser.

Diese Formulierungen sind nur für die nichtinvasive Verabreichung von Arzneistoffen in Form von liposomenartigen, den Arzneistoff enthaltenden Tröpfchen geeignet. Auch hier bestehen die bereits beschriebenen Stabilitätsprobleme und damit Einschränkungen für den Vertrieb als Fertigarzneimittel.

Der Erfindung lag die Aufgabe zugrunde, ein pharmazeutisch anwendbares System in Form eines Fertigarzneimittels zur Verfügung zu stellen, das sowohl die parenterale als auch die enterale, dermale und buccale Verabreichung von in Wasser und lipophilen Medien praktisch unlöslichen Arzneistoffen ermöglicht.

Bei der Lösung der Aufgabe war je nach Anwendung zu beachten:
1. parenterale Anwendung:
   a) intravenös
      um für eine relevante Beurteilung der Toxizität und der pharmakokinetischen Eigenschaften ausreichend hohe Blutspiegel zu erhalten, war es erforderlich, die extrem geringe Löslichkeit in Wasser und lipophilen Medien praktisch unlöslicher Arzneistoffe (< 1 mcg/100 ml) im Blut unter Ausschluß von Rekristallisationen, d.h. Bildung unkontrollierter, großer Feststoffteilchen bzw. Agglomeraten, die zu Verstopfungen der Kapillargefäße führen (können), zu verbessern,
   b) subcutan/intramuskulär
      die bekannt schlechte lokale Verträglichkeit in Wasser und lipophilen Medien praktisch unlöslicher Arzneistoffe war zu verbessern.
2. enterale Anwendung:
   a) die bekannt schlechte Wirkstoffresorption aufgrund der geringen Löslichkeit von in Wasser und lipophilen Medien praktisch unlöslicher Arzneistoffe war zu verbessern,
   b) die spontane Rekristallisation von in Wasser und lipophilen Medien praktisch unlöslichen Arzneistoffen aus hydrophilen mit Magen-/Darmsaft mischbaren Lösungen nach Vermischung mit Magen-/Darmsaft war zu vermeiden.
3. dermale Anwendung:
   der bekannt niedrige Konzentrationsgradient bei der Hautpenetration von in Wasser und lipophilen Medien praktisch unlöslichen Arzneistoffen bei dermaler Anwendung war zu vergrößern.
4. buccale Anwendung:
   die bekannt niedrige Adsorption/Resorption von in Wasser und lipophilen Medien praktisch unlöslichen Arzneistoffen bei buccaler Anwendung (z.B. Spül- und Gurgellösungen) war zu vergrößern.

Die beschriebene Aufgabe wird dadurch gelöst, daß eine stabile Lösung enthaltend den in Wasser und lipophilen Medien praktisch unlöslichen Arzneistoff, einen oder mehrere physiologisch verträgliche, in Wasser lösliche oder in Wasser micell-kolloidal lösliche Ampho-Surfactant(s) und ein oder mehrere physiologisch verträgliche, wasserfreie und wassermischbare Lösungsmittel gefunden wurde, die nach Vermischen mit Wasser eine vorübergehend stabile (metastabile) micell-kolloidale Dispersion bildet, d.h. die für die parenterale und enterale Applikation geeignete Verabreichungsform.

Die wasserfrei Arzneistoffzubereitung wird im folgenden als Dispersionskonzentrat (DC) bezeichnet.

Unter einem lipophilen Medium wird z.B. ein Fettsäuretriglycerid verstanden.

In Wasser und lipophilen Medien praktisch unlöslich bedeutet, daß die Löslichkeit des Arzneistoffes in Wasser, bzw. einem lipophilen Medium, geringer als 0,001 % ist, vorzugsweise geringer als 0,00001 %.

Unter einem in Wasser löslichen oder in Wasser micell-kolloidal löslichen Ampho-Surfactant ist ein natürliches Ampho-Surfactant zu verstehen.

Ein Ampho-Surfactant ist ein in Wasser löslicher oder in Wasser micell-kolloidal löslicher, natürlicher, physiologisch verträglicher Ampholyt, analog zum körpereigenen "Surfactant" (vgl. Mutschler, E., Arzneimittelwirkungen, 6.Auflage Stuttgart 1991 und Keidel, Wolf-Dieter, Physiologie, 6. Auflage Stuttgart - New York, 1988).

Micell-kolloidal bedeutet, daß sich bei Wasserzugabe sowohl Micellen als auch Kolloide ausbilden (0,1 bis 500 nm).

Als physiologisch verträgliche, wasserfreie und wassermischbare Lösungsmittel kommen aliphatische Alkohole, Glycerinketal, Lactame, N-Alkyl-Lactame, Aminosäurealkylester, Aminoalkoholacylester u.a. infrage.

Die Erfindung betrifft daher eine Arzneistoffzubereitung zur parenteralen, enteralen, dermalen oder buccalen Applikation enthaltend einen in Wasser und lipophilen Medien praktisch unlöslichen Arzneistoff, dadurch gekennzeichnet, daß der Arzneistoff in einer Lösung aus einem oder mehreren physiologisch verträglichen, in Wasser löslichen oder in Wasser micell-kolloidal löslichen Ampho-Surfactant/s und einem oder mehreren physiologisch verträglichen, wasserfreien und wassermischbaren Lösungsmittel/n gelöst ist.

Die erfindungsgemäße Lösung ist ein sogenanntes Dispersionskonzentrat und bildet nach Mischen mit Wasser, Blutplasma, Gewebsflüssigkeit oder Verdauungssäften spontan (ohne Energiezufuhr) metastabile, micell-kolloidale Dispersionen, die den in Wasser und lipophilen Medien praktisch unlöslichen Arzneistoff micell-kollodial gelöst oder kolloiddispers verteilt enthalten.

Je nach Applikationsart ist die Lösung unverdünnt oder nach Zusatz von Wasser, das gegebenenfalls Puffersubstanzen, Geschmackskorrigentien, ein mit Wasser in jedem Verhältnis mischbares, physiologisch verträgliches Lösungsmittel und/oder ein in Wasser lösliches physiologisch verträgliches, hydrophiles Kolloid enthält, als Verabreichungsform geeignet.

Die Erfindung betrifft weiter ein Verfahren zur Herstellung der Arzneistoffzubereitung, das dadurch gekennzeichnet ist, daß man den in Wasser und lipophilen Medien praktisch unlöslichen Arzneistoff in der wasserfreien Lösung des/der Ampho-Surfactant/s und dem/den wasserfreien und wassermischbaren Lösungsmittel(n) bei Raumtemperatur oder auch geringfügig höherer Temperatur unter Rühren löst.

Bei der Durchführung des Verfahrens ist Sauerstoffausschluß zweckmäßig, d.h. das Verfahren wird z.B. unter N₂-Schutzgas durchgeführt.

Geringfügig höhere Temperatur bedeutet Temperaturen bis 50°C, im besonderen bis 30°C.

Bei der Durchführung des erfindungsgemäßen Verfahrens entsteht eine klare Lösung, die physikalisch lagerstabil ist, d.h. die Lösung enthält auch nach längerer Standzeit (> 1 Jahr) unter den üblichen Lagerbedingungen (+ 5°C, + 23°C, + 40°C) keine Arzneistoff- oder Hilfsstoffausscheidungen.

Das erfindungsgemäße Dispersionskonzentrat enthält vorzugsweise 1 bis 20 % Arzneistoff, 1,0 bis 50 %, insbesondere 3,75 bis 27,5 % Ampho-Surfactant und Lösungsmittel ad 100 % (die Prozentangaben sind Gewichtsprozente).

Je nach Erfordernis (d.h. Dosierung, Löslichkeit der Wirk- und Hilfsstoffe) werden die Anteile der Wirkstoffe, Ampho-Surfactants und Lösungsmittel in den angegebenen Konzentrationen variiert.

Die erfindungsgemäße Arzneistoffzubereitung (= Arzneiform) ermöglicht, in Wasser und lipophilen Medien praktisch unlösliche Arzneistoffe in Form von klaren, lagerstabilen Flüssigkeiten in den Handel zu bringen (vergl. Tabelle 1).

Die erfindungsgemäße klare Lösung des in Wasser und lipophilen Medien praktisch unlöslichen Arzneistoffes wird für eine parenterale Anwendung (i.v. - i.m. - s.c.) vor der Injektion mit einer vorbestimmten Menge Wasser, das gegebenenfalls die genannten weiteren Zusätze enthält, verdünnt. Durch mehrmaliges leichtes Schütteln werden die beiden Flüssigkeiten gemischt. Dadurch entsteht die eigentliche homogene parenterale Verabreichungsform (Analogie zu "Parenteralia diluenda", Deutsches Arzneibuch 9). Die Verdünnung erfolgt in Abhängigkeit von Wirkstoffkonzentration im Dispersionskonzentrat, erforderlicher Wirkstoffdosis und dem Applikationsvolumen. Nach Zusatz von Wasser entsteht aus der klaren Lösung (DC) spontan eine homogene Dispersion von schwacher Opaleszenz. Die Dispersion ist über die für Parenteralia diluenda geforderte Zeitspanne bis zur Injektion (mindestens 2 Stunden) stabil, d. h. es scheiden sich weder Arzneistoff noch Hilfsstoff(e) aus der parenteralen Verabreichungsform, der micell-kolloidalen Dispersion, ab. (vgl. Tabelle 2).

Das erfindungsgemäße, klare Dispersionskonzentrat wird, falls eine enterale Applikation infrage kommt, beispielsweise um eine Monodosis zu erhalten, mittels bekannter Verfahren in Hart- oder Weichgelatinekapseln abgefüllt und eingenommen. Eine Verabreichung der Lösung auf z.B. Zucker ist auch möglich. Die erforderliche Menge an Dispersionskonzentrat kann auch in Trinkampullen abgefüllt und nach Zusatz von Wasser getrunken werden.

Das erfindungsgemäße klare Dispersionskonzentrat wird, falls eine dermale Applikation infrage kommt, in für die dermale Applikation geeignete Behälter, z.B. eine Glasflasche mit aufgesetzter Dosierpumpe oder eine Kunststoffquetschflasche, z.B. aus Polyethylen, abgefüllt und gemäß der für den Wirkstoff vorgesehenen gültigen Dosiervorschriften tropfenweise auf das vorgesehene Hautareal aufgebracht und leicht in die Haut einmassiert.

Das erfindungsgemäße klare Dispersionskonzentrat wird, falls eine buccale Applikation infrage kommt, in für die buccale Applikation geeignete Behälter, z.B. eine Glasflasche mit aufgesetztem Tropfer oder aufgesetzter Dosierpumpe oder Kunststoff-Monodosisbehälter, z.B. aus Polyethylen, abgefüllt und gemäß der für den Wirkstoff vorgesehenen gültigen Dosiervorschriften ohne Zusatz von Wasser oder auch nach Zusatz von Wasser in den Mundraum eingebracht, und so als Spül- bzw. Gurgellösung eingesetzt mit dem Ziel der buccalen Adsorption/Resorption des Arzneistoffes.

Als Ampho-Surfactant werden in Wasser lösliche oder in Wasser micell-kolloidal lösliche, spezielle Phospholipidgemische natürlichen Ursprungs (Soja oder Ei) mit einem Gehalt an Phosphatidylcholin von 20 bis 90 %, vorzugsweise von 40 bis 80 %, eingesetzt.

Außerdem können diese Phospholipidgemische noch Phosphatidylethanolamin, Phosphatidyl-serin, Phosphatidyl-inositol, Phosphatidyl-glycerol und freie Phosphatidsäuren enthalten, wie z.B. die Handelsprodukte Epicholin 75®, Phospholipon 50®, Lipoid S75®.

Prinzipiell können auch synthetische und halbsynthetische Phosphatide eingesetzt werden sofern sie die beschriebenen Lösungseigenschaften besitzen.

Als physiologisch verträgliche, wasserfreie und wassermischbare Lösungsmittel kommen ein- oder mehrwertige aliphatische Alkohole (z.B. Ethanol, 1,1-Iminodi-2-propanol bzw. 1,2-Propandiol) und/oder Etheralkohole (z.B. Tetrahydrofurfurylalkohol-polyethylenglykolether (z.B. Glycofurol 75®)) und/oder Glycerinketal (z.B. 2-Dimethyl-4-oxymethyl-1,3-dioxalan (wie ®Solketal) und/oder Lactame (z.B. 2-Ketopyrrolidine wie ®Soluphor P) und/oder N-Alkyl-lactame (z.B. N-Methylpyrrolidon) und/oder N-Acyl-Aminosäurealkylester, N-Acyl-Aminoalkoholacylester, Acyl-Aminoalkoholester oder das entsprechende Quaternisierungsprodukt, Aminosäurerealkylester infrage. Vorzugsweise werden ein- oder mehrwertige aliphatische Alkohole insbesondere 1,2-Propandiol und Tetrahydrofufurylalkohol-polyethylenglykolether (z.B. Glycofurol 75 und Tetraglykol®) eingesetzt.

Als Arzneistoffe kommen alle zur enteralen, parenteralen, dermalen und buccalen Anwendung geeignete Substanzen in Betracht, die in Wasser und lipophilen Medien praktisch unlöslich sind (Löslichkeit vorzugsweise geringer als 0,00001 %). Insbesondere kommen in Betracht:
1. Anti-Aids-Mittel, wie Proteaseinhibitoren, z.B. N,N*-Bis-<(2S-(1,1-Dimethylethyl-sulfonylmethyl)-3-(1-naphthyl)-propionyl)-L-valyl>-2S,5S-diamino-1,6-diphenyl-hexan-3R,4R-diol (auch als HBY 793 bezeichnet) und
   4-Isopropyloxycarbonyl-5-methoxy-3-methylthiomethyl-3,4-dihydrochinoxalin-2(1H)-thion (auch als HBY 097 bezeichnet)
2. Antirheumatika und Antipsoriatika, wie Leflunomid, 2-Cyano-3-hydroxy-N-(4-trifluormethyl-phenyl)-crotonamid und 2-Cyano-3-hydroxy-3-cyclopropyl-N-(3-methyl-4-trifluormethyl-phenyl)-propenamid
3. Antimykotika, wie Glotrimazol, Miconazol, Econazol, Bifonazol, Rilopirox, Ketoconazol, und Itraconazol.
4. Antidiabetika, wie Glibenclamid, Glimepirid.
5. Diuretika, wie Furosemid, Piretanid.

Als Arzneistoffe kommen ferner insbesondere auch hydrolyseempfindliche Arzneistoffe, beispielsweise Arzneistoffe gentechnologischen Ursprungs, infrage, z.B. Hirudin und seine entsprechend wirksamen Strukturanaloga.

Weitere Hilfsstoffe können in der Arzneistoffzubereitung enthalten sein, sie müssen sich in dem wasserfreien, wassermischbaren Lösungsmittel lösen.

Als weitere Hilfsstoffe kann das Dispersionskonzentrat z.B. hydrophile Kolloide, insbesondere Polyvinylpyrrolidon (z.B. ®Kollidon-Typen) beispielsweise in einer Menge von 1 bis 10 %, enthalten.

Weitere geeignete Hilfsstoffe sind außer den bereits erwähnten hydrophilen Kolloiden z.B. nichtionische, oberflächenaktive Stoffe, z.B. Polyethylenglykolglycerol-Triricinoleat (z.B. ®Cremophor EL) und/oder ionische oberflächenaktive Stoffe, z.B. 1,4-Bis(2-ethylhexyl)-sulfosuccinat-Na (z.B. ®Aerosol OT).

Falls der Zusatz von weiteren Hilfsstoffen zweckmäßig ist, wird das erfindungsgemäße Verfahren wie folgt durchgeführt:
Das/die wasserfreie(n) und wassermischbare(n) Lösungsmittel werden mit dem Ampho-Surfactant und den übrigen Hilfsstoffen, wozu auch das hydrophile Kolloid rechnet, homogen gemischt und gelöst; anschließend wird der in Wasser und lipophilen Medien praktisch unlösliche Arzneistoff hierin unter Rühren/Rollen ebenfalls gelöst.

Für die wasserfreien und wassermischbaren Dispersionskonzentrate sind in der Regel keine weiteren Hilfsstoffe zwingend erforderlich. Daher ist die Substanzbelastung bei enteraler, dermaler und buccaler Gabe, insbesondere aber auch nach Verdünnung mit Wasser bei parenteraler oder auch buccaler Gabe, äußerst gering.

Die wasserfreien und wassermischbaren Zubereitungen sind physiologisch einwandfrei, sie sind dosiergenau applizierbar und lagerstabil (vgl. Tabelle 2).

Zur weiteren Erläuterung der Erfindung werden folgende Beispiele genannt:

### Beispiel 1: Herstellung Dispersionskonzentrat (DC) (Allgemeine Vorschrift)

Die erfindungsgemäße Arzneistoffzubereitung (DC) besteht aus den Komponenten Arzneistoff (= in Wasser und lipophilen Medien praktisch unlöslich), dem Hilfsstoff A (= wasserfreies, mit Wasser in jedem Verhältnis mischbares Lösungsmittel), dem Hilfsstoff B (= in Wasser lösliches oder in Wasser micell-kolloidal lösliches Ampho-Sufactant), und ggfls. dem Hilfsstoff C (= in Wasser löslicher nichtionischer oder ionischer oberflächenaktiver Stoff) und/oder ggfls. dem Hilfsstoff D (= in Wasser lösliches hydrophiles Kolloid).

Hilfsstoff A wird in einem zur Herstellung von Lösungen geeigneten Ansatzkessel vorgelegt und unter Rühren der Hilfsstoff B portionsweise eingetragen. Je nach Temperatur (max. + 50°C) und Effizienz des Misch- und Rührwerkzeuges entsteht innerhalb 1-2 Stunden eine klare Lösung.

Danach werden bei Raumtemperatur ggfls. die Hilfsstoffe C und/oder D schrittweise zugesetzt. Nach ca. 1 Stunde Rühren entsteht die klare Hilfsstoff-Lösung (HSL).

In die HSL wird der Arzneistoff eingetragen. Nach kurzer Zeit entsteht eine klare Lösung, das DC.

Danach erfolgt eine Druckfiltration bei 2-2,5 bar durch eine Polyamid-Membran mit einer Porenweite von 0,2 µm. Anschließend wird in die Primärverpackungen entsprechend der Appliaktionsart abgefüllt. Im Falle der parenteralen Applikation wird das DC in Einzeldosisbehältnisse abgefüllt und im Endbehältnis (= Injektionsflasche) sterilisiert (20 Min. 121°C).

Alle Arbeiten erfolgen unter weitgehendem Sauerstoffausschluß (N₂-Schutzgas).

| Beispiel | 2 | 3 | 4 | 5 |
|---|---|---|---|---|
| Komponenten DC [mg/g] | | | | |
| Arzneistoff: HBY 793 | 10,00 | 10,00 | 40,00 | 40,00 |
| A: 1,2-Propandiol | 740,00 | - | - | - |
| A: Glycofurol 75 | - | 865,00 | 835,00 | 710,00 |
| B: Epicholin 75 | 250,00 | 125,00 | 125,00 | 250,00 |

Die Arzneistoffzubereitung DC ist lagerstabil. Die Tabelle 1 zeigt die Arzneistoff- und Klarstabilität von Formulierungen unterschiedlicher Konzentrationen und unterschiedlichen Herstellungsverfahren und Belastung im Endbehältnis.

**Tabelle 1**

| Arzneistoffstabilität | | | |
|---|---|---|---|
| Beispiel | 3 | 4 | 4 (sterilisiert 20 Min., 121°C) |
| Arzneistoffgehalt (HPLC) DC [mg/100 mg] nach 6 Monaten | | | |
| Ausgangswert | 1,01 | 4,13 | 4,18 |
| KS | - | - | 3,98 |
| RT | 1,00 | 4,05 | 3,98 |
| + 40°C | - | 4,02 | 3,97 |
| Klarstabilität | | | |

Unter allen Lagerbedingungen (6 Monate Kühlschrank (KS), Raumtemperatur (RT), 40°C) bleiben die Dispersionskonzentrate klar.

### Beispiel 6

### Herstellung der parenteralen Verabreichungsform (Allgemeine Vorschrift)

Die in Einzeldosisbehältnisse abgefüllte Arzneistoffzubereitung (DC) - Herstellung nach Beispiel 1 - wird für die parenterale Applikation mit einem vorgeschriebenen Volumen Wasser in die eigentliche Verabreichungsform überführt.

Aus dem klaren DC konstituiert sich nach mehrmaligem leichten Schütteln mit dem vorgeschriebenen Volumen Wasser rasch eine metastabile, micell-kolloidale Dispersion mit einem mittleren Teilchendurchmesser von ca. 150 nm und einer Größenverteilung im Bereich von 80 bis 250 nm.

Je nach Anwendungskonzentration und nach Zusammensetzung des DC schwankt der pH-Wert im Bereich von 5,6 bis 6,4. Die Osmolalität liegt um 500 mOsmol/kg.

Die Verabreichungsform (= micell-kolloidale Dispersion) entspricht den Anforderungen für "Injektionslösungen" oder "Infusionslösungen" gemäß DAB 10 (DAB = Deutsches Arzneibuch).

Die Tabelle 2 zeigt die chemische und physikalische Stabilität nach 6 Stunden Stehen bei Raumtempertur.

Die Dispersionskonzentrate der Beispiele 7, 8 und 9 wurden gemäß Beispiel 1 hergestellt und gemäß Beispiel 6 in die Verabreichungsformen der Beispiele 7, 8 und 9 überführt.

**Tabelle 2**

| | Beispiel 7 | Beispiel 8 | Beispiel 9 |
|---|---|---|---|
| Komponente | DC bzw. Verabreichungsform [mg/Fl.] | DC bzw. Verabreichungsform [mg/Fl.] | DC bzw. Verabreichungsform [mg/Fl.] |
| Arzneistoff: HBY 793, 95,7 %ig | 5,73 | 22,96 | 22,96 |
| B: Epicholin 75 | 69,50 | 69,50 | 69,50 |
| A: Glycofurol 75 | 480,77 | 463,54 | 463,54 |
| DC (mg/Fl.) | 556,00 | 556,00 | 556,00 |
| Wasser | 5000,00 | 5000,00 | 2222,75 |
| Verabreichungsform | 5556,00 (= 1 mg/ml) | 5556,00 (= 4 mg/ml | 2778,75 (= 8 mg/ml) |

| Wirkstoffgehalt (HPLC) [mg/Fl.] | | | |
|---|---|---|---|
| 0 Stunden | 5,89 | 23,56 | 22,78 |
| n. 6 Stunden | 5,83 | 23,56 | 22,92 |

| Mittlerer Teilchendurchmesser [nm, RT]* | | | |
|---|---|---|---|
| 0 Stunden | 149,2 | 123,9 | 165,8 |
| n. 6 Stunden | 151,4 | 127,3 | 167,6 |

| Polydispersität [RT] | | | |
|---|---|---|---|
| 0 Stunden | 0,248 | 0,287 | 0,293 |
| n. 6 Stunden | 0,178 | 0,290 | 0,241 |

| | | | |
|---|---|---|---|
| *) gemessen mit Photonen-Korrelationsspektrometer (Autosizer 2c, Fa. Malvern) | | | |
| Fl bedeutet Flasche/Endbehälter | | | |

Die Tabelle 3 zeigt die Auswertung pharmakokinetischer Untersuchungen nach intravenöser und subcutaner Verabreichung der Beispiele 7 und 8 in der Maus.

Die Untersuchungen wurden wie folgt durchgeführt:
Die Präparate wurden weiblichen, ca. 6 Wochen alten NMRI-Mäusen intravenös, subkutan oder oral mittels Schlundsonde appliziert. Zu bestimmten Zeitpunkten wurde bei je 2 oder 3 Mäusen unter Narkose eine Herzpunktion durchgeführt. Die Narkose erfolgte durch intraperitoneale Applikation von 0,3 ml einer Urethanlösung (0,2 mg/ml). Das entnommene Blut wurde bis zur Gerinnung bei 4°C aufbewaht und anschließend zentrifugiert. Das so gewonnene Serum wurde zur Reinigung nochmals zentrifugiert (Eppendorff-Zentrifuge Modell 5414,5 min). Bis zur Analyse wurde das Serum bei -20°C gelagert.

Die Analyse der Proben erfolgte mittels HPLC, wobei für die Analyse von HBY 793 eine Etherextraktion vorgeschaltet wurde. Der Gehalt wurde durch Fluorometrie bestimmt.

**Tabelle 3**

| Applikationsart | Beispiel | Dosis [mg/kg] | AUC [ngxh/ml] | AUC/Dos. | t/2 [min.] |
|---|---|---|---|---|---|
| i.v. | 7 | 8,4 | 2932 | 301 | 221 |
| i.v. | 8 | 50,0 | 6699 | 134 | 78 [std.] |
| s.c. | 7 | 4,1 | 347 | 84 | 2,2 |
| s.c. | 8 | 16,3 | 469 | 28 | 2,9 |
| AUC = Aerea under the curve | | | | | |

Die Tabelle 3 zeigt, daß bei s.c. - Gabe keine vollständige Resorption erfolgt (Normierung: i.v. - Gabe = 100 % Bioverfügbarkeit).

Die lokale Verträglichkeit ist unter allen Prüfbedingungen gut.

Analog Beispiel 1 werden die Dispersionskonzentrate der Beispiele 10 bis 14 hergestellt.

| Beispiel | 10 | 11 | 12 | 13 |
|---|---|---|---|---|
| Komponenten DC [mg/g] | | | | |
| Arzneistoff: HBY 793 | 10,00 | 10,00 | 10,00 | 30,00 |
| A: 1,2-Propandiol | 490,00 | 865,00 | - | - |
| A: Glycofurol 75 | - | - | 928,00 | 845,00 |
| B: Epicholin 75 | 250,00 | 62,50 | 46,50 | 62,50 |
| C: PEG-7-Glyceryl-cocoat | 250,00 | - | - | - |
| C: Cremophor EL | - | 62,50 | 15,50 | 62,50 |

| Beispiel | 14 | 15 | 16 | 17 |
|---|---|---|---|---|
| Komponenten DC [mg/g] | | | | |
| Arzneistoff: HBY 793 | 40,00 | 40,00 | 40,00 | 50,00 |
| A: Glycofurol 75 | 785,00 | 797,50 | 723,00 | 725,00 |
| B: Epicholin 75 | 125,00 | 62,50 | 93,500 | 125,00 |
| C: Cremophor EL | - | - | 93,50 | - |
| D: Kollidon 17 (PVP) | 50,00 | 100,00 | 50,00 | 100,00 |

Die Tabelle 4 zeigt die Auswertung pharmakokinetischer Untersuchungen von Formulierungen mit Hilfsstoffen der Gruppe C) und D) nach intravenöser Verabreichung in der Maus (Versuchsbeschreibung vergl. Untersuchungen der Beispiel 7 und 8). Die Wirkstoffkonzentration (C) wurde ermittelt nach 60 Minuten.

**Tabelle 4**

| Applikationsart | Beispiel | Dosis [mg/kg] | C 60 Min. [ng/ml] | AUC [ngxh/ml] |
|---|---|---|---|---|
| i.v. | 10 | 9,9 | 196 | 851 |
| i.v. | 13 | 29,7 | 8049 | 6297 |
| i.v. | 17 | 32,5 | 1073 | - |
| Die lokale Verträglichkeit ist gut. | | | | |

Die Tabelle 5 zeigt die pharmakokinetischen Auswertungen von Formulierungen mit unterschiedlichen Arzneistoff/Hilfsstoffverhältnissen nach subcutaner Verabreichung in der der Maus (Versuchsbeschreibung vergl. Untersuchungen der Beispiele 7 und 8).

**Tabelle 5**

| Beispiel | Arzneistoff/Hilfsstoffverh. [Teile] Arzn.: B): D) | Dosis [mg/kg] | AUD [ngxh/ml] | AUD/Dosis |
|---|---|---|---|---|
| 4 | 1 : 3,125 : - | 20 | 1818 | 90,9 |
| 15 | 1 : 1,560 : 2,5 | 20 | 1815 | 92,6 |
| 5 | 1 : 6,250 : - | 40 | 3627 | 90,7 |
| 15 | 1 : 1,560 : 2,5 | 33 | 3219 | 96,7 |
| AUD = Aerea under the data | | | | |

Die Herstellung der Disperisonskonzentrate der Beispiele 18 bis 24 erfolgte analog Beispiel 1.

| Beispiel | 18 | 19 | 20 | 21 |
|---|---|---|---|---|
| Komponenten DC [mg/g] | | | | |
| Arzneistoff: HBY 097 | 20,00 | 20,00 | 20,00 | 50,00 |
| A: Glycofurol 75 | 855,00 | 792,50 | 817,50 | 762,50 |
| B: Epicholin 75 | 125,00 | 93,75 | 62,50 | 93,75 |
| C: Cremophor EL | - | 93,75 | - | 93,75 |
| D: Kollidon 17 (PVP) | - | - | 100,00 | - |

| Beispiel | 22 | 23 | 24 |
|---|---|---|---|
| Komponenten DC [mg/g] | | | |
| Arzneistoff: HBY 097 | 50,00 | 100,00 | 200,00 |
| A: 1,2-Propandiol | 762,50 | - | - |
| A: Glycofurol 75 | - | 618,75 | 518,75 |
| B: Epicholin 75 | 93,75 | 93,75 | 93,75 |
| C: Cremophor EL | 93,75 | - | 187,50 |
| C: Cremophor RH 40 | - | 187,50 | - |

Die Tabelle 6 zeigt pharmakokinetische Auswertungen von unterschiedlichen Formulierungen mit dem Arzneistoff HBY 097 an unterschiedlichen Tierarten und unterschiedlichen Applikationsarten.

Die Untersuchungen an der Maus wurden wie unter den Beispielen 7 und 8 angegeben durchgeführt. Am Hund wurden die Tests wie folgt durchgeführt.

### Hund:

Männliche Hunde (Beagles), Körpergewicht ca. 20 kg, wurden ca. 16 Stunden vor Versuchsbeginn in Einzelkäfige gesetzt. Bis zum Ende der Versuchs erhielten die Tiere kein Futter, während Trinkwasser unbegrenzt zur Verfügung stand. Für die orale Applikation wurde den Hunden das Prüfpräparat jeweils in Kapseln verabreicht, die tief in den Rachen geschoben wurden. Mit Hilfe einer 20 ml Spritze wurde mit ca. 20 ml Leitungswasser nachgespült.

Die intravenöse Gabe von Prüfpräparaten erfolgte durch Injektion in die Vena cephalica antebrachii, wobei darauf geachtet wurde, daß dieselbe Vene im Versuch nicht zur Entnahme von Blutproben benutzt wurde.

Zu den vorgesehenen Zeitpunkten wurden durch Punktion der Vena cephalica antebrachii je 5 - 8 ml Blut in heparinisierten Probenröhrchen aufgefangen. Die Proben wurden im Eisbad für ca. 1 Stunde aufbewaht und anschließend zentrifugiert (10 min. 3000 rpm bei 4°C). Das heparinisierte Plasma wurde zur Reinigung nochmals zentrifugiert und anschließend bei -20°C bis zur Analyse aufbewahrt.

### Analyse

Die Analyse der Proben erfolgte mittels HPLC, wobei für die Analyse von HBY 097 eine Fällung mit Acetonitril vorgeschaltet wurde. Der Gehalt wurde durch Messung der UV-Absorption bestimmt.

**Tabelle 6**

| Tierart | Applikationsart | Beispiel | Dosis [mg/kg] | AUC [ngxh/ml] |
|---|---|---|---|---|
| Maus | i.v. | 19 | 7,4 | 668 |
| | | | 16,0 | 2703 |
| | p.o. | 22 | 100,0 | 2322 |
| | | | 100,0 | 4275 |
| Hund | i.v. | 23 | 5,3 | 6188 |
| | i.v. | 19 | 1,7 | 925 |
| | | | 2,0 | 956 |

Analog Beispiel 1 wurden die Dispersionskonzentrate der Beispiele 25 bis 41 hergestellt.

| Beispiel | 25 | 26 | 27 | 28 |
|---|---|---|---|---|
| Komponenten DC [mg/g] | | | | |
| Rilopirox, dermal | 10,00 | 10,00 | - | - |
| Leflunomid, dermal | - | - | 10,00 | 10,00 |
| A: 1,2-Propandiol | - | - | 740,00 | 740,00 |
| A: Glycofurol 75 | 865,00 | 740,00 | - | - |
| B: Epicholin 75 | 125,00 | - | 250,0 | 187,50 |
| B: Lipoid S 75 | - | 187,50 | - | - |
| C: Cremophor RH 40 | - | 62,50 | - | 62,50 |

| Beispiel | 29 | 30 | 31 | 32 | 33 | 34 |
|---|---|---|---|---|---|---|
| Komponenten DC [mg/g] | | | | | | |
| Glimepirid, p.o. | 10,0 | 15,0 | - | - | - | - |
| Itraconazol, i.v. | - | - | 5,0 | 5,0 | 5,0 | 5,0 |
| A: 1,2-Propandiol | 807,5 | - | - | - | - | - |
| A: Glycofurol 75 | - | 685,0 | 745,0 | 770,0 | 870,0 | 682,5 |
| B: Epicholin 75 | - | - | 250,0 | 125,0 | 125,0 | 125,0 |
| B: Phospholipon 50^{R} | 62,5 | - | - | - | - | - |
| B: Lipoid S 75 | - | 125,00 | - | - | - | - |
| C: Cremophor RH 40 | - | 125,00 | - | - | - | 187,5 |
| C: Myrij 52 | 95,00 | - | - | - | - | - |
| C: Dioctyl-sulfo-succinat | 25,00 | - | - | - | - | - |
| D: Kollidon 17 (PVP) | - | 50,00 | - | 100,00 | - | - |

| Beispiel | 35 | 36 | 37 |
|---|---|---|---|
| Komponenten DC [mg/g] | | | |
| 2-Cyano-3-hydroxy-N-(4- | | | |
| trifluormethyl-phenyl)-crotonamid | 10,0 mg | 50,0 mg | 50,0 mg |
| A: 1,1-Iminodi-2-propanol | - | 50,0 mg | 50,0 mg |
| A. Glycofurol 75 | 740,0 mg | - | - |
| A: 1,2-Propandiol | - | 775,0 mg | 650,0 mg |
| B: Lipoid S 75 | 250,0 mg | 125,0 mg | 250,0 mg |

| Beispiel | 38 | 39 | 40 | 41 |
|---|---|---|---|---|
| Komponenten DC [mg/g] | | | | |
| Furosemid | 15,0 mg | 10,0 mg | - | - |
| Piretanid | - | - | 5,0 mg | 5,0 mg |
| A:Glycofurol 75 | 685,0 mg | 740,0 mg | 770,0 mg | 870,0 mg |
| B: Epicholin 75 | - | 250,0 mg | 125,0 mg | 125,0 mg |
| B: Lipoid S 75 | 125,0 mg | - | - | - |
| C: Cremophor RH 40 | 125,0 mg | - | - | - |
| D: Kollidon 17/PVP | 50,0 mg | - | 100,0 mg | - |

## Patentansprüche

1. Arzneistoffzubereitung zur parenteralen, enteralen, dermalen oder buccalen Applikation enthaltend einen in Wasser und lipophilen Medien praktisch unlöslichen Arzneistoff, dadurch gekennzeichnet, daß der Arzneistoff in einer Lösung aus einem oder mehreren physiologisch verträglichen, in Wasser löslichen oder in Wasser micell-kolloidal löslichen Ampho-Surfactant(s) und einem oder mehreren physiologisch verträglichen, wasserfreien und wassermischbaren Lösungsmittel(n) gelöst ist.

2. Arzneistoffzubereitung gemäß Anspruch 1, dadurch gekennzeichnet, daß sie weitere in dem wasserfreien, wassermischbaren Lösungsmittel lösliche, physiologisch verträgliche Hilfsstoffe enthält.

3. Arzneistoffzubereitung gemäß Anspruch 1, dadurch gekennzeichnet, daß sie zusätzlich ein physiologisch verträgliches, hydrophiles Kolloid, und/oder physiologisch verträgliche, nichtionische oder ionische, oberflächenaktive Substanzen enthält.

4. Arzneistoffzubereitung gemäß Anspruch 1, dadurch gekennzeichnet, daß das Ampho-Surfactant ein Phospholipidgemisch mit einem Phosphatidylcholingehalt von 40 bis 80 % ist und das Lösungsmittel 1,2-Propandiol oder Tetrahydrofurfurylalkohol-polyethylenglycolether ist.

5. Arzneistoffzubereitung gemäß Anspruch 1, dadurch gekennzeichnet, daß der Arzneistoff ein Anti-Aids-Mittel, ein Antirheumatikum, ein Antipsoriatikum, ein Diuretikum, ein Antimykotikum oder ein Antidiabetikum ist.

6. Wasserhaltige Dispersion zur parenteralen, enteralen oder buccalen Applikation von in Wasser und lipophilen Medien praktisch unlöslichen Arzneistoffen, dadurch gekennzeichnet, daß die Arzneistoffzubereitung gemäß einem der Ansprüche 1 bis 5 zusätzlich Wasser, das gegebenenfalls physiologisch verträgliche Puffersubstanzen, Geschmackskorrigentien, ein mit Wasser in jedem Verhältnis mischbares, physiologisch verträgliches Lösungsmittel und/oder ein in Wasser lösliches, physiologisch verträgliches, hydrophiles Kolloid enthält, aufweist.

7. Verfahren zur Herstellung einer Arzneistoffzubereitung gemäß Anspruch 1, dadurch gekennzeichnet, daß man den in Wasser und lipophilen Medien praktisch unlöslichen Arzneistoff in der wasserfreien Lösung des/der Ampho-Surfactant/s und dem/den wasserfreien und wassermischbaren Lösungsmittel(n) bei Raumtemperatur oder auch geringfügig höherer Temperatur löst.

8. Verfahren zur Herstellung einer wasserhaltigen Dispersion gemäß Anspruch 6, dadurch gekennzeichnet, daß man eine Arzneistoffzubereitung gemäß einem der Ansprüche 1 bis 5 in Wasser, das gegebenenfalls physiologisch verträgliche Puffersubstanzen, Geschmackskorrigentien, ein mit Wasser in jedem Verhältnis mischbares, physiologisch verträgliches Lösungsmittel und/oder ein in Wasser lösliches, physiologisch verträgliches, hydrophiles Kolloid enthält, dispergiert.
